(19) Europäisches Patentamt
European Patent Office
Office européen des brevets

(11) Veröffentlichungsnummer: 0 163 234
B1

(12) EUROPÄISCHE PATENTSCHRIFT

(45) Veröffentlichungstag der Patentschrifft: 28.02.90

(51) Int. Cl. 5: C 07 C 47/02, C 07 C 45/50, C 07 C 47/225

(21) Anmeldenummer: 85106184.6

(22) Anmeldetag: 21.05.85

(54) **Verfahren zur Herstellung von Aldehyden.**

(30) Priorität: 01.06.84 DE 3420491

(43) Veröffentlichungstag der Anmeldung: 04.12.85 Patentblatt 85/49

(45) Bekanntmachung des Hinweises auf die Patenterteilung: 28.02.90 Patentblatt 90/09

(84) Bennante Vertragsstaaten: AT DE FR GB IT NL SE

(56) Entgegenhaltungen:
FR-A-2 314 910
FR-A-2 473 504
FR-A-2 489 308
US-A-3 928 232

**Die Akte enthält technische Angaben, die nach dem Eingang der Anmeldung eingereicht wurden und die nicht in dieser Patentschrift enthalten sind.**

(73) Patentinhaber: HOECHST AKTIENGESELLSCHAFT
Postfach 80 03 20
D-6230 Frankfurt am Main 80 (DE)

(72) Erfinder: Bahrmann, Helmut, Dr. Dipl.-Chem.
Rohstrasse 48
D-4236 Hamminkeln (DE)
Erfinder: Cornils, Boy, Dr. Dipl.-Chem.
Friedrich-Ebert-Strasse 45
D-4220 Dinslaken (DE)
Erfinder: Konkol, Werner, Dr. Dipl.-Chem.
Lützowstrasse 40a
D-4200 Oberhausen 11 (DE)
Erfinder: Lipps, Wolfgang, Dr. Dipl.-Chem.
Falkestrasse 76
D-4200 Oberhausen 11 (DE)

EP 0 163 234 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

**Beschreibung**

Die vorliegende Erfindung betrifft ein Verfahren zur Herstellung von Aldehyden durch Hydroformylierung von Olefinen in Gegenwart wasserlöslicher Rhodium-Komplexkatalysatoren.

Es ist bekannt, durch Umsetzung von Olefinen mit Kohlenmonoxid und Wasserstoff Aldehyde und Alkohole herzustellen. Die Reaktion wird durch Hydridometallcarbonyle, vorzugsweise solcher der Metalle der 8. Gruppe des Periodensystems, katalysiert. Neben Kobalt, das als Katalysatormetall in großem Umfang technische Anwendung findet, gewinnt in letzter Zeit Rhodium zunehmende Bedeutung. Im Gegensatz zu Kobalt gestattet Rhodium, die Reaktion bei niedrigem Druck durchzuführen, darüber hinaus werden vorzugsweise geradkettige n-Aldehyde und nur in untergeordnetem Maße iso-Aldehyde gebildet. Schließlich ist auch die Hydrierung der Olefine zu gesättigten Kohlenwasserstoffen bei Anwendung von Rhodium-Katalysatoren deutlich niedriger als bei Anwendung von Kobalt-Katalysatoren.

Bei den in der Technik eingeführten Verfahren wird der Rhodium-Katalysator in Form von modifizierten Hydridorhodiumcarbonylen eingesetzt, die zusätzliche und gegebenenfalls überschüssige Liganden enthalten. Besonders bewährt haben sich als Liganden tertiäre Phosphine oder Phospite.

Ihre Anwendung ermöglicht, den Reaktionsdruck auf Werte unter 300 bar ($3 \cdot 10^4$ kPa) zu senken.

Probleme wirft bei diesem Verfahren jedoch die Abtrennung der Reaktionsprodukte und die Wiedergewinnung der im Reaktionsprodukt homogen gelösten Katalysatoren auf. Im allgemeinen destilliert man hierzu das Umsetzungsprodukt aus dem Reaktionsgemisch ab. In der Praxis kann dieser Weg wegen der thermischen Empfindlichkeit der gebildeten Aldehyde und Alkohole aber nur bei der Hydroformylierung niedriger Olefine, d.h. Olefine mit bis zu etwa 5 Kohlenstoffatomen in Molekül, beschritten werden. Außerdem hat sich gezeigt, daß die thermische Belastung des Destillationsgutes auch zu erheblichen Katalysatorverlusten durch Zersetzung der Rhodiumkomplexverbindungen führt.

Die geschilderten Mängel werden durch Anwendung von Katalysatorsystemen vermieden, die in Wasser löslich sind. Derartige Katalysatoren sind z. B. in der DE-PS-2 627 354 beschrieben. Die Löslichkeit der Rhodiumkomplexverbindungen wird hierbei durch Verwendung von sulfonierten Triarylphosphinen als Komplexbestandteil erreicht. Die Abtrennung des Katalysators vom Reaktionsprodukt nach Beendigung der Hydroformylierungsreaktion erfolgt bei dieser Verfahrensvariante einfach durch Trennung von wässriger und organischer Phase, d.h. ohne Destillation und damit ohne zusätzliche thermische Verfahrensschritte. Ein weiteres Merkmal dieser Arbeitsweise ist, daß mit hoher Selektivität aus endständigen Olefinen n-Aldehyde und nur in ganz untergeordnetem Maße iso-Aldehyde gebildet werden. Neben sulfonierten Triarylphosphinen werden als Komplexbestandteile wasserlöslicher Rhodiumkomplexverbindungen auch carboxylierte Triarylphosphine eingesetzt.

Die bekannten Verfahren haben sich ausgezeichnet bei der Hydroformylierung niederer Olefine, insbesondere Ethylen und Propylen, bewährt. Setzt man höhere Olefine wie Hexen, Octen oder Decen ein, gehen der Umsatz und/oder die Selektivität zu n-Verbindungen merklich zurück. Die Wirtschaftlichkeit der Umsetzung in technischem Maßstab ist dann häufig nicht mehr gegeben.

Der Rückgang des Umsatzes wird durch die Abnahme der Löslichkeit höherer Olefine in Wasser verursacht, denn die Reaktion zwischen den Reaktanten läuft in der wässrigen Phase ab.

Es ist zwar aus der DE-3 135 127 Al bekannt, die Hydroformylierung olefinischer Verbindungen bei Vorliegen von wässriger und mit ihr nicht oder nur wenig mischbarer organischer Phase in Gegenwart von Lösungsvermittlern vorzunehmen.

Die praktische Durchführung dieser Umsetzung ist ausschließlich auf den Einsatz monosulfonierter bzw. monocarboxylierter Triarylphosphine als Bestandteil der Rhodiumkomplexverbindung beschränkt. Dabei zeigt sich, daß insbesondere das monosulfonierte Triphenylphosphin nur zu einem mäßigen Umsatz führt und die Selektivität zu geradkettigen n-Aldehyden gering ist.

Umsatz und Selektivität lassen sich dadurch verbessern, daß man trisulfonierte Triarylphosphine statt der monosulfonierten Verbindungen verwendet. Unbefriedigend ist bei dieser Verfahrensvariante jedoch, daß - wenn auch in geringen Mengen - Rhodium und wasserlösliches Phosphin mit dem organischen Reaktionsprodukt ausgetragen werden, so daß in vielen Fällen ein zusätzlicher Aufarbeitungsschritt erforderlich wird. Ein weiterer Nachteil ist die damit verbundene Abnahme des n/i-Verhältnisses.

Es bestand daher die Aufgabe, die vorstehend geschilderten Nachteile zu überwinden und eine Arbeitsweise zu entwickeln, die es erlaubt, auch höhere Olefine in einem aus wäßriger Katalysatorlösung und organischen Ausgangsstoffen und gegebenenfalls Reaktionsprodukten sowie gasförmigen Reaktanten bestehenden Mehrphasensystem zu hydroformylieren.

Erfindungsgemäß wird die vorstehend beschriebene Aufgabe gelöst durch ein Verfahren zur Herstellung von Aldehyden durch Umsetzung von Olefinen mit 6 bis 20 Kohlenstoffatomen mit Kohlenmonoxid und Wasserstoff in flüssiger Phase in Gegenwart von Wasser sowie Rhodium in metallischer Form oder als Verbindung und eines wasserlöslichen Arylphosphins bei Temperaturen von 20 bis 150°C und 1 bis 200 bar (100 bis $2 \cdot 10^4$ kPa). Es ist dadurch gekennzeichnet, daß das wasserlösliche Phosphin der allgemeinen Formel

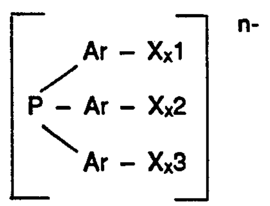
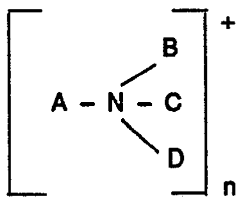

folgt, wobei Ar für eine Arylgruppe und X für eine Sulfonsäuregruppe steht, $x^1$, $x^2$, $x^3$ 0 oder 1 bedeutet mit der Maßgabe, daß mindestens eine Zahl $x^1$, $x^2$ oder $x^3$ 1 ist, A für einen Alkyl- oder Aralkylrest mit 7 bis 18 Kohlenstoffatomen steht und B, C, D geradkettige oder verzweigte Alkylreste mit 1 bis 4 Kohlenstoffatomen sind, n ist eine ganze Zahl zwischen 1 und 3.

Überraschenderweise hat sich gezeigt, daß bei Einsatz der wasserlöslichen Phosphine nach dem erfindungsgemäßen Verfahren auch bei der Hydroformylierung höherer Olefine die hohe Aktivität und Selektivität des Katalysatorsystems erhalten bleiben. Gleichzeitig wird aber auch die mit dem organischen Reaktionsprodukt ausgetragene Phosphinmenge erheblich reduziert.

Die nach dem neuen Verfahren eingesetzten wasserlöslichen Phosphine fördern offensichtlich die Löslichkeit des organischen Substrats in der wässrigen Phase und tragen so zur Erhöhung des Umsatzes bei. Ihre äußerst geringe Löslichkeit in der organischen Phase hat zur Folge, daß sie selbst und die Metallkomponente des Katalysatorsystems nicht oder nur in vernachlässigbar kleiner Menge zusammen mit dem Reaktionsprodukt aus der Reaktionszone ausgetragen wird. Ein gesonderter Aufarbeitungsschritt zur Rückgewinnung von Rhodium aus dem Aldehyd erübrigt sich daher.

Unter den wasserlöslichen Phosphinen der oben wiedergegebenen allgemeinen Formel werden im Rahmen der neuen Arbeitsweise insbesondere Verbindungen eingesetzt, in denen Ar eine Phenyl- oder Naphthylgruppe, die Summe von $x^1$, $x^2$ und $x^3$ 2 oder 3 und B, C und D die gleichen geradkettigen oder verzweigten Alkylgruppen mit 1 bis 4 Kohlenstoffatomen bedeuten.

Beispiele für wasserlösliche Phosphine, die sich zur Durchführung des neuen Verfahrens eignen, sind Triphenyltrisulfonate und Triphenyldisulfonate mit folgenden Kationen: Trimethylcetylammonium, Trimethyldodecylammonium, Tributyldodecylammonium, Dodecylethyl-dimethylammonium, Triethylbenzylammonium.

Zur Herstellung der in dem beanspruchten Verfahren verwendeten Phosphine geht man von sulfonierten Triarylphosphinen aus, die durch Behandlung von Triarylphosphinen mit Oleum erhalten werden. Durch Variation der Reaktionsbedingungen, insbesondere der Reaktionszeit, der Reaktionstemperatur und des Verhältnisses von Triarylphosphin zu Schwefeltrioxid lassen sich mono-, di- oder trisulfonierte Arylphosphine herstellen.

Zweckmäßig gewinnt man aus dem Sulfonierungsprodukt zunächst in Wasser unlösliche, in organischen Lösungsmitteln jedoch lösliche Aminsalze. Sie werden anschließend durch Behandlung mit einem quartären Ammoniumhydroxid in das gewünschte "Onium"-Salz des sulfonierten Triarylphosphins überführt.

Die Umsetzung des Olefins mit Wasserstoff und Kohlenmonoxid nach dem neuen Verfahren erfolgt bei Temperaturen von 20 bis 150°C, insbesondere 50 bis 120°C und Drücken von 1 bis 200 bar (100 bis $2 \cdot 10^4$ kPa), insbesondere 10 bis 100 bar ($1 \cdot 10$ bis $1 \cdot 10^4$ kPa).

Der Katalysator kann dem Reaktionssystem präformiert zugesetzt werden. Mit gleich gutem Erfolg läßt er sich aber auch aus den Komponenten Rhodium oder Rhodiumverbindung und der wässrigen Lösung des quartären Ammoniumsalzes des sulfonierten Triarylphosphins unter Reaktionsbedingungen im Reaktionsgemisch, also in Gegenwart des Olefins, herstellen. Außer metallischem Rhodium in feinverteilter Form können als Rhodiumquelle wasserlösliche Rhodiumsalze wie Rhodiumchlorid, Rhodiumsulfat, Rhodiumacetat oder in organischen Medien lösliche Verbindungen wie Rhodium-2-ethylhexanoat oder unlösliche Verbindungen wie Rhodiumoxide eingesetzt werden.

Die Rhodiumkonzentration in der wäßrigen Katalysatorlösung beträgt 10 bis 2000 Gew.-ppm, bezogen auf die Lösung. Das quartäre Ammoniumsalz des sulfonierten Phosphins wird in einer solchen Menge eingesetzt, daß auf 1 Grammatom Rhodium 1 bis 300 Mol, vorzugsweise 2 bis 100 Mol, Phosphinverbindung kommen.

Der pH-Wert der wäßrigen Katalysatorlösung soll nicht unter 2 liegen. Allgemein stellt man einen pH-Wert von 2 bis 13, vorzugsweise 4 bis 10, ein.

Die Zusammensetzung des Synthesegases, d.h. das Verhältnis von Kohlenmonoxid zu Wasserstoff, kann in weiten Grenzen variiert werden. Im allgemeinen setzt man ein Synthesegas ein, in dem das Volumverhältnis von Kohlenmonoxid zu Wasserstoff 1 : 1 beträgt oder von diesem Wert nur wenig abweicht.

Die Umsetzung kann sowohl absatzweise als auch kontinuierlich durchgeführt werden.

Das erfindungsgemäße Verfahren wird mit Erfolg bei der Hydroformylierung von geradkettigen oder verzweigten Olefinen mit sechs bis zwanzig Kohlenstoffatomen angewandt. Die Doppelbindung in diesen Olefinen kann end- oder innenständig sein.

Die folgenden Beispiele erläutern die Erfindung näher ohne sie auf die beschriebenen Ausführungsformen zu beschränken. Zur Charakterisierung der Leistungsfähigkeit der Katalysatorsysteme wird neben dem Verhältnis von n-Aldehyd zu i-Aldehyd der Begriff der "Aktivität" definiert als

$$\frac{\text{mol Aldehyde}}{\text{g-Atom Rh} \cdot \text{min}}$$

verwendet. Die Alkohol- und Kohlenwasserstoffbildung ist minimal.

**Beispiel 1 (Vergleich)**

In einem 1 l Autoklaven mit Tauchstutzen werden 420 g (entsprechend 355 ml) einer wässrigen Lösung, die 15,5 Gew.% Tri(m-sulfophenyl)phosphin-Na-Salz enthält, sowie 400 ppm Rh als Rh-Acetat vorgelegt. Darauf wird Synthesegas ($CO/H_2$ = 1 : 1) bis zu einem Druck von 25 bar aufgepreßt. Die Reaktionslösung wird bei 125°C 3 h unter Rühren mit dem Synthesegas behandelt. Man kühlt auf etwa 30°C herunter, stellt die Rührung ab und drückt nach einer Absetzzeit von 15 Minuten die überschüssige Lösung über den Tauchstutzen heraus (≈ 61 g), sie wird analysiert. Die restliche Lösung verbleibt im Autoklaven. Zu ihr werden unter Rühren über eine Druckpumpe 170 g n-Hexen-1 gepumpt. Unter Aufrechterhaltung eines Druckes von 25 bar wird 3 Stunden auf 125°C erhitzt. Danach läßt man auf 30°C abkühlen und absitzen. Die überstehende organische Phase wird nach einer 15-minütigen Beruhigungsdauer über den Tauchstutzen herausgedrückt. Die organische Phase wird gewogen und gaschromatographisch untersucht.

Die Hydroformylierung wird insgesamt sechsmal wiederholt, wobei im wesentlichen die gleichen Ergebnisse resultieren.

Die in der Tabelle 1 aufgeführten Aktivitätswerte beziehen sich auf die aktuell im Autoklaven vorhandenen Mengen wässriger und organischer Phase.

**Tabelle 1**

| Anzahl der Hydroformylierungen | 1 | 2 | 3 | 4 | 5 | 6 | 7 |
|---|---|---|---|---|---|---|---|
| Umsatz (% nach GC) | 21 | 18 | 21 | 21 | 20 | 21 | 19 |
| n/i-Verhältnis | 98/2 | 98/2 | 98/2 | 98/2 | 98/2 | 98/2 | 98/2 |
| wässrige Phase im Reaktor (g) | 359 | 359 | 359 | 357 | 357 | 357 | 356 |
| organische Phase aus Reaktor (g) | 175 | 174 | 176 | 175 | 175 | 165 | 175 |
| Aktivität $\frac{\text{mol } C_7\text{-Aldehyde}}{\text{g-Atom Rh} \cdot \text{min}}$ | 1,28 | 1,09 | 1,29 | 1,29 | 1,23 | 1,21 | 1,17 |

Zur Ermittlung der mit der organischen Phase insgesamt ausgetragenen Rhodium- und Phosphormengen werden die dem Reaktor in den einzelnen Versuchen entnommenen organischen Anteile vereinigt, auf etwa 1/10 der ursprünglichen Menge eingeengt und analysiert. Es werden 0,017 Gew.-ppm Rh und 0,34 Gew.-ppm Phosphor (jeweils bezogen auf die ursprüngliche organische Phase) gefunden.

**Beispiel 2**

Beispiel 1 wird wiederholt mit dem Unterschied, daß anstelle des Na-Salzes 315 g (entsprechend 295 ml) einer wässrigen Lösung des Trimethylbenzylammonium-salzes von Tri-(m-sulfophenyl)phosphin mit einem P(III)-Gehalt von 0,308 Gew.-% und 158 g n-Hexen-1 in die Hydroformylierung eingesetzt werden. Die Versuchsergebnisse sind in Tabelle 2 zusammengestellt.

Die Ermittlung der Rhodium- und Phosphorverluste erfolgt wie in Beispiel 1 beschrieben. Es werden durchschnittlich 0,029 Gew.-ppm Rhodium und 0,98 Gew.-ppm Phosphor über das organische Produkt ausgetragen, d.h. nur geringfügig mehr, als bei Einsatz des üblicherweise verwendeten Na-Salzes des Tri(m-sulfophenyl)phosphins.

**Tabelle 2, Beispiel 2**

| Anzahl der Hydroformylierungen | 1 | 2 | 3 | 4 | 5 | 6 | 7 | 8 |
|---|---|---|---|---|---|---|---|---|
| Umsatz (% nach GC) | 48 | 55 | 51 | 50 | 41 | 38 | 41 | 41 |
| n/i-Verhältnis | 93/7 | 93/7 | 93/7 | 94/6 | 94/6 | 94/6 | 94/6 | 94/6 |
| wäßrige Phase im Reaktor (g) | 315 | 314 | 312 | 312 | 311 | 309 | 309 | 308 |
| organische Phase aus Reaktor (g) | 180 | 198 | 182 | 155 | 164 | 160 | 158 | 178 |
| Aktivität $\frac{\text{mol } C_7\text{-aldehyde}}{\text{g-Atom Rh} \cdot \text{min}}$ | 3.43 | 4.34 | 3.72 | 3.11 | 2.71 | 2.46 | 2.62 | 2.96 |

**Beispiele 3 - 5**

Die Beispiele 3 - 5 werden entsprechend Beispiel 1 durchgeführt mit dem Unterschied, daß anstelle des Natriumsalzes-des Tri-(m-sulfophenyl)-phosphins

420 g (entsprechend 390 ml) einer wässrigen Lösung, die 46 Gew.-% des Dodecylethyldimethylammonium-salzes von Tri-(m-sulfophenyl)-phosphin enthält (Beispiel 3)

820 g (entsprechend 740 ml) einer wässrigen Lösung, die 27 Gew.-% des Benzyltrimethylammonium-salzes von Di-(m-sulfophenyl)-phenylphosphin (Beispiel 4)

420 g (entsprechend 390 ml) einer wässrigen Lösung, die 23 Gew.-% des Benzyltriethylammonium-salzes von Tri-(m-sulfophenyl)-phosphin enthält (Beispiel 5)

eingesetzt werden. Die Ergebnisse der Versuche sind in Tabelle 3 zusammengestellt.

**Tabelle 3**

| | Beispiele | | |
|---|---|---|---|
| **TPPTS-Oniumsalz** | **3** | **4** | **5** |
| Olefin | 1-Hexen | 1-Hexen | 1-Hexen |
| ø Umsatz (% nach GC) | 97 | 78 | 60 |
| ø n/i-Verhältnis | 77/23 | 96/4 | 96/4 |
| ø Aktivität $\frac{\text{mol Aldehyd}}{\text{g-Atom Rh} \cdot \text{min}}$ | 13,3 | 5,4 | 5,0 |
| Anzahl der Hydroformylierungen mit der gleichen Katalysatorlösung | 15 | 10 | 10 |

3) Dodecylethyldimethylammonium/TPPTS (420 g = 390 ml einer 46 %-igen Lösung)
4) Benzyltrimethylammonium/TPPDS (820 g = 740 ml einer 27 %-igen Lösung)
5) Benzyltriethylammonium/TPPTS (410 g = 390 ml einer 23 %-igen Lösung)

**Beispiele 6 und 7**

Auch die Beispiele 6 und 7 werden unter den Bedingungen des Beispiels 1 durchgeführt, jedoch mit Styrol als Olefin. In Beispiel 6 (Vergleichsbeispiel) wird das Na-salz (420 g entsprechend 375 ml einer 22 Gew.-% enthaltenden Lösung) in Beispiel 7 das Dodecylethyldimethylammonium-salz (420 g entsprechend 391 ml einer 23 Gew.-% enthaltenden Lösung) des Tri-(m-sulfophenyl)phosphin verwendet. Die Ergebnisse der Versuche sind Tabelle 4 zu entnehmen. Wie ersichtlich, begünstigt das quartäre Ammoniumsalz die Bildung des alpha-Phenylpropionaldehyds.

**Tabelle 4**

| | Beispiele | |
|---|---|---|
| **TPPTS-Oniumsalz** | **6** | **7** |
| Olefin | Styrol | Styrol |
| ø Umsatz (% nach GC) | 6 | 100 |
| ø0 $\beta/\alpha$-Verhältnis | 40/60 | 26/74 |
| ø Aktivität $\frac{\text{mol Aldehyd}}{\text{g-Atom Rh} \cdot \text{min}}$ | 0,2 | 6,5 |
| Anzahl der Hydroformylierungen mit der gleichen Katalysatorlösung | 5 | 5 |

6) TPPTS-Na-Salz (Vergleich) (420 g = 375 ml einer 22 %-igen Lösung)
7) Dodecylethyldimethylammonium/TPPTS (420 g = 391 ml einer 23 %-igen Lösung)

**Patentansprüche**

1. Verfahren zur Herstellung von Aldehyden durch Umsetzung von Olefinen mit 6 bis 20 Kohlenstoffatomen mit Kohlenmonoxid und Wasserstoff in flüssiger Phase in Gegenwart von Wasser sowie Rhodium in metallischer Form oder als Verbindung und eines wasserlöslichen Arylphosphins bei Temperaturen von 20 bis 150°C und 1 bis 200 bar (100 bis $2 \cdot 10^4$ kPa), dadurch gekennzeichnet, daß das wasserlösliche Phosphin der allgemeinen Formel folgt, wobei Ar füreine Arylgruppe und X für eine Sulfonsäuregruppe steht, $x^1$, $x^2$, $x^3$ 0 oder 1 bedeutet mit der Maßgabe, daß mindestens eine Zahl $x^1$, $x^2$ oder $x^3$ 1 ist, A für einen Alkyl- oder Aralkylrest mit 7 bis 18 Kohlenstoffatomen steht und B, C, D geradkettige oder verzweigte Alkylreste mit 1 bis 4 Kohlenstoffatomen sind und n eine ganze Zahl zwischen 1 und 3 ist.

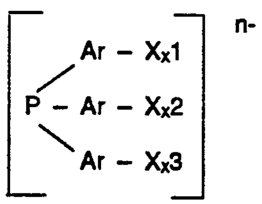

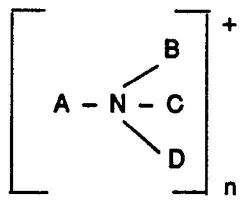

2. Verfahren nach Anspruch 1, dadurch gekennzeichnet, daß Ar die Phenyl- oder Naphthylgruppe bedeutet.

3. Verfahren nach Anspruch 1 und 2, dadurch gekennzeichnet, daß die Summe von $x^1$, $x^2$, $x^3$ 2 oder 3 ist.

4. Verfahren nach Anspruch 1 bis 3, dadurch gekennzeichnet, daß B, C und D die gleichen Alkylgruppen bedeuten.

**Claims**

1. A process for preparing aldehydes by the reaction of olefins having 6 to 20 carbon atoms with carbon monoxide and hydrogen in liquid phase in the presence of water, rhodium in metallic form or as a compound and a water-soluble arylphosphine at temperatures of 20 to 150°C and pressures of 1 to 200 bar (100 to $2 \times 10^4$ kPa), characterised in that the water-soluble phosphine has the general formula:

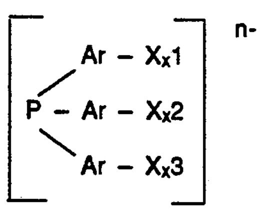

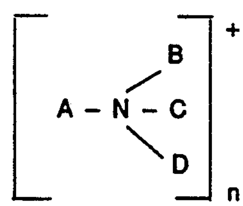

where Ar denotes an aryl group and X a sulfonic acid group, $x^1$, $x^2$ and $x^3$ are 0 or 1 subject to the condition that at least one number $x^1$, $x^2$ or $x^3$ is 1, A stands for an alkyl or, aralkyl radical having 7 to 18 carbon atoms and B, C and D are straight-chain or branched alkyl radicals having 1 to 4 carbon atoms and n is an integer between 1 and 3.

2. A process according to claim 1, characterised in that Ar stands for the phenyl or naphthyl group.

3. A process according to claims 1 and 2, characterised in that the sum of $x^1$, $x^2$ and $x^3$ is 2 or 3.

4. A process according to claims 1 to 3, characterised in that B, C and D are the same alkyl groups.

**Revendications**

1. Procédé pour la fabrication d'aldéhydes par réaction d'oléfines en $C_6$ - $C_{20}$ avec le monoxyde de carbone et l'hydrogène en phase liquide en présence d'eau et de rhodium sous forme métallique ou sous forme de composé et d'une arylphosphine soluble dans l'eau à des températures de 20 à 150°C et sous 1 à 200 bar (0,1 à 20 MPa), caractérisé en ce que la phosphine soluble dans l'eau répond à la formule générale

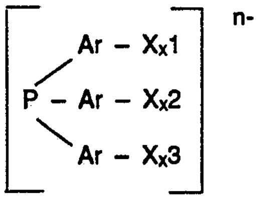

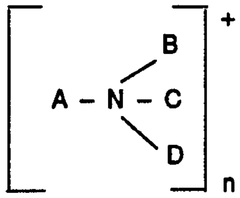

dans laquelle Ar représente un groupe aryle et X un groupe acide sulfonique, $x^1$, $x^2$ et $x^3$ représentent 0 ou 1 avec la condition que l'un au moins des nombres $x^1$, $x^2$ $x^3$ est égal à 1, A représente un reste alkyle ou arylalkyle en $C_7$ - $C_{18}$ et B, C, D représentent des restes alkyles à chaîne droite ou ramifiée en $C_1$ - $C_4$ et n est un nombre entier compris entre 1 et 3.

2. Procédé selon la revendication 1, caractérisé en ce que Ar représente le groupe phényle ou naphtyle.

3. Procédé selon les revendications 1 et 2, caractérisé en ce que la somme de $x^1$, $x^2$ et $x^3$ est égale à 2 ou 3.

4. Procédé selon les revendications 1 à 3, caractérisé en ce que B, C et D sont les mêmes groupes alkyles.